(19) Europäisches Patentamt
European Patent Office
Office européen des brevets

(11) Veröffentlichungsnummer: **0 286 889 B1**

(12) **EUROPÄISCHE PATENTSCHRIFT**

(45) Veröffentlichungstag der Patentschrift: **21.08.91**

(51) Int. Cl.5: **C07C 257/08**

(21) Anmeldenummer: **88104817.7**

(22) Anmeldetag: **25.03.88**

(54) **Verfahren zur Herstellung von ortho-substituierten Arylcarboximido-estern.**

(30) Priorität: **04.04.87 DE 3711460**

(43) Veröffentlichungstag der Anmeldung:
**19.10.88 Patentblatt 88/42**

(45) Bekanntmachung des Hinweises auf die Patenterteilung:
**21.08.91 Patentblatt 91/34**

(84) Benannte Vertragsstaaten:
**CH DE FR GB IT LI NL**

(56) Entgegenhaltungen:
**EP-A- 0 093 443**
**EP-A- 0 200 051**

(73) Patentinhaber: **HOECHST AKTIENGESELLSCHAFT**
**Postfach 80 03 20**
**W-6230 Frankfurt am Main 80(DE)**

(72) Erfinder: **Knorr, Harald, Dr.**
**Völklinger Weg 34**
**W-6000 Frankfurt am Main 71(DE)**
Erfinder: **Salbeck, Gerhard, Dr.**
**Frankfurter Strasse 34**
**W-6238 Hofheim am Taunus(DE)**

**EP 0 286 889 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Arylcarboximidoester dienen als Ausgangsmaterialien für N-Carbamylarylcarboxyimidoester, welche Verwendung als Schädlingsbekämpfungsmittel finden (EP-A 0135894).

Die Herstellung von Arylcarboximidoesterhydrochloriden durch Addition von Alkoholen an substituierte Benzonitrile ist in Chem. Rev. 61 (1961) 179 beschrieben. Bei Verwendung von ortho-substituierten Benzonitrilen als Ausgangsprodukte werden die gewünschten Imidoesterhydrochloride jedoch nur in geringen Ausbeuten erhalten (J. Austr. Chem. 25 (1972) 1997) oder es findet - beispielsweise bei Verwendung von 2-Chlorbenzonitril - keine Umsetzung statt, s. Chem. Rev. 61 (1961) 182-3; J. Chem. Soc. 83, 766 (1903). Bei Verwendung von 2-Ethoxybenzonitril werden nach A. Pinner, Chem. Ber. 23 (1890) 2942 nicht auftrennbare Gemische erhalten.

Nach DE-OS 3 514 450 werden bei Umsetzung von o-Benzonitrilen dann bessere Ergebnisse erzielt, wenn man anstelle von HCl wasserfreie Flußsäure einsetzt. Nachteilig bei dieser Arbeitsweise ist der Einsatz von toxischer Flußsäure. Beim Umgang mit dieser Substanz müssen strenge Sicherheitsvorkehrungen getroffen werden. Zusätzliche Nachteile ergeben sich auch dadurch, daß Flußsäure in Kombination mit Alkoholen nur in ganz speziellen korrosionsbeständigen Apparaturen gehandhabt werden kann und gängige VA-Stähle nicht verwendet werden dürfen.

Es ist ferner ungünstig, daß bei dem Verfahren nach DE-OS 3 514 450 mit Überschußmengen an Flußsäure gearbeitet wird. Durch Abdestillieren kann der Überschuß nur teilweise beseitigt werden, so daß bei der anschließenden Freisetzung des Imidats durch Zugabe von Alkali deutliche Mengen an Fluoriden entstehen, deren Entsorgung problematisch ist.

Überraschenderweise wurde nun gefunden, daß man ortho-substituierte Arylcarboximidoesterhydrochloride durch Umsetzung von ortho-Benzonitrilen mit Alkoholen und Chlor-oder Bromwasserstoff direkt und in guten Ausbeuten erhält, wenn die Reaktion unter Druck durchgeführt wird. Hierdurch werden die zuvor genannten Nachteile vermieden.

Gegenstand der Erfindung ist daher ein Verfahren zur Herstellung von Verbindungen der Formel I

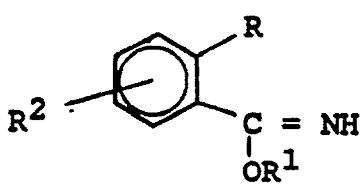

(I)

worin

R = Fluor, Chlor, Brom oder ($C_1$-$C_4$)Alkyl,
$R^1$ = ($C_1$-$C_4$)Alkyl,
$R^2$ = H, Methyl, Methoxy, Fluor, Chlor, Brom oder Trifluormethyl, vorzugsweise H, und
X = Cl oder Br bedeuten, durch Umsetzung von Nitrilen der Formel II

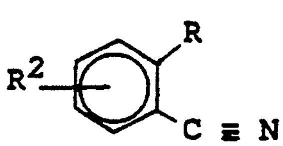

$R^1$-OH

(II) (III)

mit Alkoholen der Formel III und Chlor- oder Bromwasserstoff, dadurch gekennzeichnet, daß man die Reaktion bei Drücken von 1 bis 50 bar und Temperaturen von -10 °C bis +50 °C gegebenenfalls in Gegenwart eines Verdünnungsmittels durchführt und das erhaltene Hydrochlorid oder Hydrobromid anschließend in die freie Base überführt.

Die Umsetzung unter Druck kann auch in Gegenwart von katalytischen Mengen von Chloriden wie $PCl_5$, $SbCl_5$, $TiCl_4$, $AlCl_3$, $FeCl_3$, $SnCl_4$ oder KJ oder in Gegenwart von Alkalisulfiden durchgeführt werden.

Als Benzonitrile der Formel (II) lassen sich beispielsweise die Verbindungen 2-Fluorbenzonitril, 2-Chlorbenzonitril, 2,6-Difluorbenzonitril, 2,6-Dichlorbenzonitril, 2-Chlor-4-methylbenzonitril, 2-Brombenzonitril, 2-Methylbenzonitril, insbesondere 2-Chlor-, 2-Fluor- und 2,6-Difluorbenzonitril einsetzen.

Als Alkohole der Formel II werden bevorzugt Methanol, Ethanol, Propanol-1, Propanol-2 eingesetzt. Der Alkohol der Formel III wird zweckmäßigerweise in äquivalenten Mengen oder in einem Überschuß von bis zu 50 Mol-% bezogen auf das Nitril der Formel II verwendet. Die Alkohole der Formel III können darüberhinaus auch als Verdünnungsmittel eingesetzt werden.

Der Temperaturbereich der Umsetzung variiert insbesondere zwischen 0°C und 30°C.

Als Druck wird zweckmäßigerweise der durch das Aufpressen von gasförmigem Chlor- oder Bromwasserstoff oder durch Zugabe von flüssigem Chlor- oder Bromwasserstoff entstehende Eigendruck verstanden. Die Drücke können zwischen 1 bis 50 bar, insbesondere zwischen von 3 und 20 bar variieren.

Als Verdünnungsmittel können inerte Verbindungen verwendet werden, beispielsweise Kohlenwasserstoffe, die halogeniert sein können, z.B. Heptan, Oktan, 1.2.2-Trifluor-1.1.2 trichloräthan oder Dichlormethan, Aether, wie Diisopropyläther oder Methyl-tert.butyläther, Dioxan oder Tetrahydrofuran; Aromaten wie Toluol, Xylol, Chlorbenzol oder Trifluormethylbenzol.

Das erfindungsgemäße Verfahren läßt sich in der Weise durchführen, daß man zunächst das Nitril der Formel II zusammen mit dem Alkohol III gegebenenfalls in einem Verdünnungsmittel im Druckgefäß vorlegt und dann unter Kühlung Chlor- oder Bromwasserstoffgas aufpreßt oder flüssiges Chlor- oder Bromwasserstoff zudosiert. Es ist jedoch auch möglich, das Nitril der Formel II vorzugeben und Chlor- oder Bromwasserstoff gasförmig oder in flüssiger Form und anschließend den jeweiligen Alkohol III hinzuzufügen.

Zur Isolierung von (I) fügt man gegebenenfalls ein mit Wasser nicht mischbares Lösungsmittel, das nicht umgesetztes Nitril löst, zum anfallenden Reaktionsgemisch hinzu und trennt die Hydrochloride bzw. -bromide der Verbindungen der Formel I zweckmäßigerweise durch Extraktion mit Wasser ab. Die Verbindungen (I) werden dann aus den Hydrochloriden bzw. -bromiden durch Zugabe einer wäßrigen Base erhalten. Als Basen eignen sich Alkali- oder Erdalkalihydroxide, -carbonate oder -hydrogencarbonate wie NaOH, KOH, $Na_2CO_3$, $K_2CO_3$, $NaHCO_3$, $KHCO_3$.

Nachfolgende Beispiele dienen zur näheren Erläuterung der Erfindung.

**Beispiel 1**

Herstellung von 2-Chlorphenylcarboximidoäthylester

In einem 4 Liter Emailkessel werden 137,6 g 2-Chlorbenzonitril in 800 ml ®Frigen 113 (®Frigen 113 = 1,2,2-Trifluor-1,1,2-trichloräthan) und 50,7 g trockenem Ethanol vorgelegt. Bei Raumtemperatur drückt man unter Kühlung 511 g Chlorwasserstoffgas auf und rührt bis ca. 90 % des 2-Chlorbenzonitrils umgesetzt sind. Man entspannt den Chlorwasserstoffüberschuß, entnimmt den Kesselinhalt und fügt zuerst Wasser hinzu, trennt die organische Phase ab und destilliert das Lösungsmittel ab. Das verbliebene Nitril kann dem Folgeansatz zugeschlagen werden. Dann fügt man ®Frigen 113 und eine gesättigte Sodalösung hinzu, bis ein pH-Wert 8 erreicht ist, und trennt die organische Phase ab. Man trocknet, filtriert vom Trockenmittel ab und entfernt das Lösungsmittel. Es verbleiben 175,7 g 2-Chlorphenylcarboximidoäthylester mit einer Reinheit von 94 %; das Produkt kann in dieser Form direkt weiterverarbeitet werden.

**Vergleichsbeispiel**

137,6 g 2-Chlorbenzonitril in 800 ml ®Frigen 113 werden mit 50,7 g trockenem Ethanol vorgelegt. Bei Raumtemperatur leitet man 365 g Chlorwasserstoffgas in das Gemisch ein, wobei drucklos gearbeitet wird. Selbst nach zweitägigem Rühren des Reaktionsgemisches findet keine nennenswerte Umsetzung statt. Die Aufarbeitung wie unter Beispiel 1 beschrieben liefert 139 g eines Feststoffes, der zu 97-98 % aus 2-Chlorbenzonitril besteht.

**Patentansprüche**

**1.** Verfahren zur Herstellung von Verbindungen der Formel I

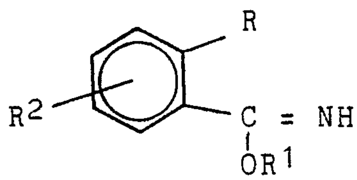

(I)

worin
R = Fluor, Chlor, Brom oder $(C_1-C_4)$Alkyl,
$R^1$ = $(C_1-C_4)$Alkyl,
$R^2$ = H, Methyl, Methoxy, Fluor, Chlor, Brom oder Trifluormethyl und
X = Cl oder Br bedeuten, durch Umsetzung von Nitrilen der Formel II

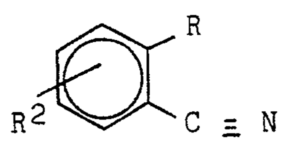

$R^1$-OH

(II) (III)

mit Alkoholen der Formel III und Chlor- oder Bromwasserstoff, dadurch gekennzeichnet, daß man die Reaktion bei Drücken von 1 bis 50 bar und Temperaturen von -10° C bis +50° C gegebenenfalls in Gegenwart eines Verdünnungsmittels durchführt und das erhaltene Hydrochlorid oder Hydrobromid anschließend in die freie Base überführt.

2. Verfahren nach Anspruch 1, dadurch gekennzeichnet, daß die Reaktionstemperatur zwischen 0° C und 30° C variiert.

3. Verfahren gemäß Ansprüchen 1 oder 2, dadurch gekennzeichnet, daß man bei Drücken von 3 bis 20 bar arbeitet.

4. Verfahren nach Ansprüchen 1, 2 oder 3, dadurch gekennzeichnet, daß man gasförmigen Chlor- oder Bromwasserstoff verwendet.

5. Verfahren nach Ansprüchen 1, 2 oder 3, dadurch gekennzeichnet, daß man flüssigen Chlor- oder Bromwasserstoff verwendet.

6. Verfahren nach Ansprüchen 1 bis 5, dadurch gekennzeichnet, daß man Chlorwasserstoff verwendet.

7. Verfahren gemäß Ansprüchen 1 bis 6, dadurch gekennzeichnet, daß man als Verbindung der Formel II 2-Chlor-, 2-Fluor- oder 2,6-Difluorbenzonitril einsetzt.

## Claims

1. A process for the preparation of a compound of the formula I

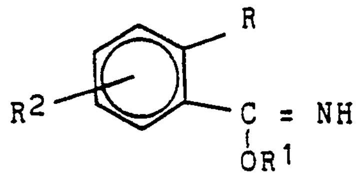

(I)

in which

- R denotes fluorine, chlorine, bromine or ($C_1$-$C_4$)alkyl,
- $R^1$ denotes ($C_1$-$C_4$) alkyl,
- $R^2$ denotes H, methyl, methoxy, fluorine, chlorine, bromine or trifluoromethyl, and
- X denotes Cl or Br, by reaction of a nitrile of the formula II

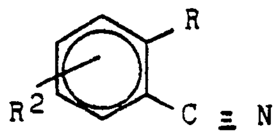

(II)

$R^1$-OH

(III)

with an alcohol of the formula III and hydrogen chloride or bromide, which comprises carrying out the reaction under pressures of 1 to 50 bar and at temperatures from -10° C to +50° C, if appropriate in the presence of a diluent, and subsequently converting the resulting hydrochloride or hydrobromide into the free base.

**2.** The process as claimed in claim 1, wherein the reaction temperature varies between 0° C and 30° C.

**3.** The process as claimed in claim 1 or 2, wherein the reaction is carried out under pressures of 3 to 20 bar.

**4.** The process as claimed in claim 1, 2 or 3, wherein gaseous hydrogen chloride or bromide is used.

**5.** The process as claimed in claim 1, 2 or 3, wherein liquid hydrogen chloride or bromide is used.

**6.** The process as claimed in claims 1 to 5, wherein hydrogen chloride is used.

**7.** The process as claimed in claims 1 to 6, wherein 2-chloro-, 2-fluoro- or 2,6-difluorobenzonitrile is used as the compound of the formula II.

## Revendications

**1.** Procédé pour préparer des composés répondant à la formule I :

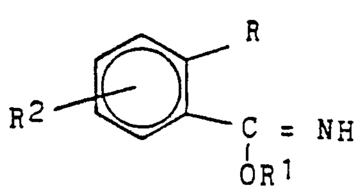

(I)

dans laquelle

- R représente le fluor, le chlore, le brome ou un alkyle en $C_1$-$C_4$,
- $R^1$ représente un alkyle en $C_1$-$C_4$,

$R^2$ représente H, un méthyle, un méthoxy, le fluor, le chlore, le brome ou un trifluorométhyle et

X représente Cl ou Br,

par réaction de nitriles de formule II avec des alcools de formule III :

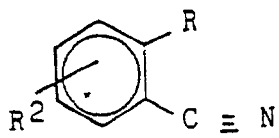

(II)

$R^1-OH$

(III)

et du chlorure ou du bromure d'hydrogène, procédé caractérisé en ce qu'on effectue la réaction sous des pressions de 1 à 50 bar et à des températures de -10 à +50° C, éventuellement en présence d'un diluant, et on transforme ensuite le chlorhydrate ou le bromhydrate obtenu en la base libre.

**2.** Procédé selon la revendication 1 caractérisé en ce que la température réactionnelle est comprise entre 0 et 30° C.

**3.** Procédé selon l'une des revendications 1 et 2 caractérisé en ce qu'on opère sous des pressions de 3 à 20 bar.

**4.** Procédé selon l'une quelconque des revendications 1, 2 et 3, caractérisé en ce qu'on utilise du chlorure ou du bromure d'hydrogène gazeux.

**5.** Procédé selon l'une quelconque des revendications 1, 2 et 3, caractérisé en ce qu'on utilise du chlorure ou du bromure d'hydrogène liquide.

**6.** Procédé selon l'une quelconque des revendications 1 à 5, caractérisé en ce qu'on utilise du chlorure d'hydrogène

**7.** Procédé selon l'une quelconque des revendications 1 à 6, caractérisé en ce qu'on utilise, comme composé de formule II, le chloro-2, fluoro-2 ou difluoro-2,6 benzonitrile.